# EUROPÄISCHE PATENTANMELDUNG

(11) **EP 2 731 035 A1**
(43) Veröffentlichungstag der Anmeldung: **14.05.2014**
(21) Anmeldenummer: 13191918.5
(22) Anmeldetag: 07.11.2013
(51) Int. Cl.: G06F 19/00

(54) **Verfahren zur selektiven Präsentation von in einer Patiententasche enthaltenen Informationen**

(30) Priorität: 09.11.2012 DE 102012110777
(71) Anmelder: Optiplan Gesellschaft für optische Planungsgeräte mit beschränkter Haftung, 40489 Düsseldorf (DE)
(72) Erfinder: Lerner, Sabine, 40489 Düsseldorf (DE); Wagner, Kai, 40489 Düsseldorf (DE)
(74) Vertreter: Stenger, Watzke & Ring

(57) **Zusammenfassung**

Die Erfindung betrifft ein Verfahren zur selektiven Präsentation von in einer Patiententasche enthaltenen Informationen, welche zumindest Informationen über noch zu leistende pflegerische und/oder medizinische Verrichtungen an einem Patienten aufweisen. Um die Führung einer Patiententasche auch mittels eines Computers zu ermöglichen, wobei dem Arzt oder der Pflegekraft stets ein aktueller Status des Patienten gegeben wird, schlägt die Erfindung vor, dass die Informationen patientenbezogen in ein oder mehrere, in einem Datenspeicher eines Computers gespeicherte, Formulare eingetragen werden, wobei die an dem Patienten bereits geleisteten Verrichtungen in einem die jeweilige Verrichtung betreffenden Formular eingetragen werden, wobei eine auf dem Computer gespeicherte Funktion automatisch auf die in den Formularen eingetragenen Informationen zugreift und selektiv die noch zu leistenden Verrichtungen mittels mehrerer auf einem Bildschirm des Computers dargestellter Anzeigeelemente anzeigt.

## Beschreibung

Die Erfindung betrifft ein Verfahren zur selektiven Präsentation von in einer Patiententasche enthaltenen Informationen, welche zumindest Informationen über noch zu leistende pflegerische und/oder medizinische Verrichtungen an einem Patienten aufweisen.

Die sogenannten Patiententaschen werden seit langer Zeit in Krankenhäusern oder ähnlichen pflegerischen Einrichtungen verwendet, um Informationen über einen Patienten wie beispielsweise dessen persönliche Daten oder auch seinen medizinischen Zustand zu sammeln und dem Arzt und/oder dem Pflegepersonal unter anderem während der Visite beim Patienten zur Verfügung zu stehen. Die Patiententasche weist dabei eine Vielzahl von Formularen auf, mit welchen die Informationen über Untersuchungsergebnisse, Medikationen und durchgeführte und/oder noch durchzuführende pflegerische Maßnahmen dokumentiert werden. Damit der Benutzer der Patiententasche, das heißt beispielsweise der Arzt oder die Pflegekraft, schnell eine Zusammenfassung aus der Patiententasche erhält, so dass er oder sie nicht kostbare Zeit durch das Blättern in der Patiententasche verliert, sind im Stand der Technik sogenannte Signalleisten bekannt, die aus einzelnen Anzeigeelementen bestehen. Diese zumeist farblich gekennzeichneten Anzeigeelemente haben die Aufgabe, dem jeweiligen Benutzer die Hinweise zu geben, welche er benötigt, insbesondere diejenigen pflegerischen und/oder medizinischen Verrichtungen anzuzeigen, die an dem Patienten noch nicht geleistet wurden und somit unerledigte Maßnahmen darstellen. In der Praxis erfolgt das Setzen der Signale so, dass ein einem bestimmten Formular zugeordnetes Anzeigeelement beispielsweise über den Seitenrand des Formulars herausgestellt wird, um dem Benutzer die noch zu erledigende Verrichtung auf einen Blick anzuzeigen. Sobald die entsprechende Verrichtung am Patienten geleistet wurde, wird das Anzeigeelement zurückgesetzt. Zu diesem Zweck weist jedes Formularblatt ein bewegliches Anzeigeelement auf, welches von einer hervorstehenden Position in eine zurückgestellte Position bewegbar ist. Dieses Zurückstellen kann mittels einer Schiebebewegung erfolgen.

Um der fortschreitenden Unterstützung der Ärzte und Pflegekräfte durch Computer Rechnung zu tragen, werden die vorgenannten Patientendaten zunehmend auch auf Computern, insbesondere Laptops, gespeichert, welche der zuständige Arzt oder auch die Pflegekraft beim Besuch des Patienten mitführen kann.

Dabei liegt der vorliegenden Erfindung die Aufgabe zugrunde, eine virtuell auf dem Computer geführte Patiententasche mit einer den Inhalt zusammenfassenden Funktionalität zu versehen, so dass der Arzt die auf dem Computer gespeicherten Patienteninformationen schnell und leicht optisch erfassen kann.

Zur Lösung der vorgenannten Aufgabe schlägt die Erfindung vor, dass die Informationen patientenbezogen in ein oder mehrere, in einem Datenspeicher eines Computers gespeicherte, Formulare eingetragen werden, wobei die an dem Patienten bereits geleisteten Verrichtungen in einem die jeweilige Verrichtung betreffenden Formular eingetragen werden, wobei eine auf dem Computer gespeicherte Funktion automatisch auf die in den Formularen eingetragenen Informationen zugreift und selektiv die noch zu leistenden Verrichtungen mittels mehrerer auf einem Bildschirm des Computers dargestellter Anzeigeelemente anzeigt.

Mittels des vorgenannten Verfahrens wird es somit möglich, dem Arzt oder der Pflegekraft eine Zustandsübersicht über eine auf einem Computer geführte Patiententasche zu geben. Zu diesem Zweck wird auf einem Computer, welchen der Arzt oder die Pflegekraft beispielsweise auch bei einer Visite mitführen kann, eine Patiententasche für jeden einzelnen Patienten angelegt. Diese Patiententasche enthält gespeicherte Formulare, in welche Daten über den Patienten eingetragen werden. Diese Daten können neben Daten zur Identität der Person ebenfalls Informationen zu dem medizinischen Zustand des Patienten umfassen. Auf jedem Formularblatt der virtuellen Patiententasche kann eine oder mehrere Informationen erfasst werden, beispielsweise pflegerische Verrichtungen, diagnostische Maßnahmen und Anordnungen, Informationen und Fragen an den Arzt, Messungen von Temperatur, Puls und/oder Blutdruck, Blutentnahmen, Röntgenleistungen, Operationen, Infusionen, Medikationen, Informationen vom behandelnden Arzt an die Pflegekräfte und so weiter. Jede dieser Informationen ist dabei einem Anzeigeelement des jeweiligen virtuellen Formulars, auf dem die Information eingetragen ist, zugeordnet. Die Verrichtungen im Sinne der vorliegenden Erfindung umfassen dabei nicht nur tatsächliche Verrichtungen von pflegerischen oder auch ärztlichen Leistungen, sondern ebenfalls auch empfangsbedürftige Informationen von dem Arzt an die Pflegekraft oder umgekehrt oder auch weitere aktuelle Informationen wie beispielsweise Medikationsänderungen.

Sobald eine entsprechende Information empfangen wurde, eine noch durchzuführende Verrichtung durchgeführt wurde und so weiter trägt die jeweilige Person die Erledigung in das jeweilige Formularblatt ein. Auf diese Zustandsinformationen greift sodann eine auf dem Computer vorhandene Funktion, das heißt ein entsprechendes Computerprogramm, automatisch zu, um den aktuellen Zustand der jeweiligen Verrichtung aus dem Formular zu extrahieren und in einer Zustandsübersicht darzustellen, welche die geleisteten und/oder noch zu leistenden Verrichtungen selektiv mittels mehrerer auf einem Bildschirm des Computers dargestellter Anzeigeelemente anzeigt.

Die Anzeigeelemente können dabei neben der Art der einzelnen zu leistenden Verrichtungen ebenfalls Informationen über den jeweiligen Wochentag, an dem die Verrichtung durchzuführen ist, sowie eine bestimmte Uhrzeit, welche insbesondere bei zeitkritisch zu verabreichenden Medikamenten wichtig ist, enthalten.

Im Sinne der Erfindung ist dabei vorteilhaft jeder Art von Verrichtung ein Anzeigeelement zugeordnet. Somit kann der Arzt oder auch die Pflegekraft unmittelbar eine Information darüber erhalten, welche Verrichtung noch auszuführen ist, ohne ergänzend in der Patiententasche blättern zu müssen.

Besonders vorteilhaft ist das jeweilige Anzeigeelement so mit dem korrespondierenden Formular verknüpft, dass der Computer einem Benutzer bei Anwahl des jeweiligen Anzeigeelementes alle auf dem korrespondierenden Formular eingetragenen Informationen auf dem Bildschirm anzeigt. Somit ergibt sich für den Benutzer im Wesentlichen die gleiche Funktionalität wie bei einer in Papierform geführten Patiententasche. Die Anzeigeelemente geben dem Benutzer einen Hinweis darauf, welche Verrichtungen aktuell noch durchzuführen sind. Sofern er weitere Informationen dazu benötigt, klickt er das entsprechende Anzeigeelemente an, woraufhin alle auf dem Formular eingetragenen Informationen angezeigt werden.

Vorteilhaft werden die Anzeigeelemente als mehrere unmittelbar zueinander benachbarte Indexstreifen angeordnet. Dadurch entsteht eine Signalleiste, welche einen Extrakt der wichtigsten Informationen aus der Patiententasche darstellt.

Besonders vorteilhaft weisen die Anzeigeelemente dabei eine der jeweiligen Information zugeordnete Farbe auf. So kann beispielsweise die Farbe Schwarz für eine pflegerische Verrichtung stehen, die noch auszuführen ist, während die Farbe Orange für diagnostische Maßnahmen steht, die Farbe Blau für Informationen, die der Arzt an das Pflegepersonal richtet und so weiter. Somit kann der Betrachter, welcher mit der Farbcodierung vertraut ist, schnell auf einen Blick den aktuellen Zustand überblicken.

Vorteilhaft kann dabei die Anzahl der noch zu leistenden Verrichtungen zusätzlich zu den vorgenannten Anzeigeelementen als Ziffer dargestellt werden. Dabei zeigt beispielsweise die Ziffer 4 an, dass der Arzt und/oder die Pflegekräfte noch vier Verrichtungen wie beispielsweise Medikamentengabe, Temperaturmessung und so weiter verrichten müssen.

Die Erfindung sieht weiterhin vor, dass die an einem Patienten bereits geleisteten Verrichtungen in einem die jeweilige Verrichtung betreffenden Formular eingetragen werden, wodurch ein dem jeweiligen Formular zugeordnetes Anzeigeelement nach der Eintragung von einem farbigen in einen grau unterlegten Zustand wechselt. Alternativ kann bei Verrichtungen, die zum Beispiel zweimal täglich ausgeführt werden müssen, ein dem jeweiligen Formularblatt zugeordnetes Anzeigeelement nach der Eintragung der ersten Verrichtung zur Hälfte von einem farbigen in einen grau unterlegten Zustand wechseln. Dadurch erhält der zuständige Arzt oder die zuständige Pflegekraft die Information, dass diese Verrichtung noch ein weiteres Mal durchzuführen ist.

Obwohl die Erfindung zuvor anhand eines Verfahrens zur Erstellung einer Zusammenfassung aus einer Patiententasche erläutert wurde, ist im Sinne der Erfindung ebenfalls ein Computerprogrammprodukt miteinbezogen, mittels welchem sich das Verfahren zur Erstellung der Zusammenfassung ausführen lässt. Darüber hinaus wird in die Erfindung ausdrücklich auch ein Computer mit einem Computerprogramm einbezogen, welches zur Ausführung des erfindungsgemäßen Verfahrens geeignet ist.

Im Folgenden wird ein Ausführungsbeispiel der Erfindung näher erläutert:

Für die Durchführung des erfindungsgemäßen Verfahrens werden auf einer Station eines Krankenhauses oder einer entsprechenden Einrichtung Patiententaschen als Dateien auf einem Computer gespeichert. Dabei enthält jede Patiententasche Formulare, in welche patientenbezogene Daten wie beispielsweise der Name und die Adresse des Patienten, das Alter, Größe und Gewicht, durchgeführte Operationen, aktuelle Medikation, Beschwerden, Messwerte von Körpertemperatur, Puls oder Blutdruck und so weiter eingetragen werden. Zu jeder Art von Verrichtung ist ein separates Formular vorgesehen, auf welchem jeweils nur die aktuelle Medikation oder aktuelle Messwerte von Temperatur, Puls, Blutdruck und so weiter eingetragen werden.

Eine auf dem Computer vorhandene Funktion greift auf die in den Formularen eingetragenen aktuellen Zustandswerte und Informationen zu, welche eine Auskunft über die geleisteten und/oder noch zu leistenden Verrichtungen geben. In Abhängigkeit dieser aktuellen Zustandsinformationen werden die Anzeigeelemente automatisch als mehrere unmittelbar zueinander benachbarte Indexstreifen dargestellt, welche dem Betrachter, das heißt dem Arzt oder der Pflegekraft, schnell und übersichtlich eine selektive Information über die aktuell noch durchzuführenden Verrichtungen geben. Dabei werden die noch durchzuführenden Verrichtungen in Abhängigkeit ihrer Art mit einer bestimmten Farbe gekennzeichnet. Beispielsweise steht die Farbe Rosa für eine aktuell noch durchzuführende Temperatur- und Pulskontrolle. Die Farbe Türkis zeigt an, dass eine Blutdruckkontrolle noch erfolgen muss. Die Pflegekraft erkennt diese Information anhand der Farbigkeit der Anzeigeelemente und führt die entsprechende Temperatur, Puls- und Blutdruckkontrolle durch. Anschließend trägt sie die an dem Patienten bereits geleisteten Messungen in dem die jeweilige Verrichtung betreffenden Formular ein, wodurch das zugeordnete Anzeigeelement von dem farbigen, das heißt rosanen oder türkisen, Zustand in einen grau unterlegten Zustand wechselt. Sofern es sich bei der entsprechenden Messung um eine solche handelt, die beispielsweise zweimal täglich durchzuführen ist, wechselt die Farbigkeit des Anzeigeelements nach der Durchführung der ersten Messung zur Hälfte in einen grau unterlegten Zustand.

Somit reduzieren sich folglich nach der Durchführung einer Verrichtung die noch farbig dargestellten Anzeigeelemente.

Sofern der Computer, auf welchem das vorgenannte Verfahren durchgeführt wird, mit anderen Computern der Station oder auch des gesamten Krankenhauses vernetzt ist, erhalten mehrere zuständige Ärzte oder Pflegekräfte gleichzeitig einen aktuellen Zustandsbericht des Patienten. Dabei kann vorgesehen sein, dass die an dem Netzwerk angeschlossenen Computer miteinander synchronisiert werden.

Somit ermöglicht die Erfindung insgesamt eine schnelle und unkomplizierte Erfassung des Patientenzustandes, wobei dem Benutzer stets automatisch ein selektiver Extrakt aus der Patiententasche als Zustandsübersicht gegeben wird.

## Patentansprüche

1. Verfahren zur selektiven Präsentation von in einer Patiententasche enthaltenen Informationen, welche zumindest Informationen über noch zu leistende pflegerische und/oder medizinische Verrichtungen an einem Patienten aufweisen,
**dadurch gekennzeichnet,**
**dass** die Informationen patientenbezogen in ein oder mehrere, in einem Datenspeicher eines Computers gespeicherte, Formulare eingetragen werden, wobei die an dem Patienten bereits geleisteten Verrichtungen in einem die jeweilige Verrichtung betreffenden Formular eingetragen werden, wobei eine auf dem Computer gespeicherte Funktion automatisch auf die in den Formularen eingetragenen Informationen zugreift und selektiv die noch zu leistenden Verrichtungen mittels mehrerer auf einem Bildschirm des Computers dargestellter Anzeigeelemente anzeigt.

2. Verfahren nach Anspruch 1, **dadurch gekennzeichnet, dass** jeder Art von Verrichtung ein Anzeigeelement zugeordnet ist.

3. Verfahren nach Anspruch 1 oder 2, **dadurch gekennzeichnet, dass** das jeweilige Anzeigeelement so mit dem korrespondierenden Formular verknüpft ist, dass der Computer einem Benutzer bei Anwahl des jeweiligen Anzeigeelementes alle auf dem korrespondierenden Formular eingetragenen Informationen auf dem Bildschirm anzeigt.

4. Verfahren nach einem der vorhergehenden Ansprüche, **dadurch gekennzeichnet, dass** die Anzeigeelemente als mehrere unmittelbar zueinander benachbarte Indexstreifen angeordnet werden.

5. Verfahren nach einem der vorhergehenden Ansprüche, **dadurch gekennzeichnet, dass** jedes Anzeigeelement eine der jeweiligen Information zugeordnete Farbe aufweist.

6. Verfahren nach einem der vorhergehenden Ansprüche, **dadurch gekennzeichnet, dass** ein dem jeweiligen Formular zugeordnetes Anzeigeelement nach der Eintragung von einem farbigen in einen grau unterlegten Zustand wechselt.

7. Verfahren nach einem der vorhergehenden Ansprüche, **dadurch gekennzeichnet, dass** ein dem jeweiligen Formular zugeordnetes Anzeigeelement bei einer zweimal täglich zu leistenden Verrichtung nach der Eintragung der ersten Verrichtung zur Hälfte von einem farbigen in einen grau unterlegten Zustand wechselt.

8. Verfahren nach einem der vorhergehenden Ansprüche, **dadurch gekennzeichnet, dass** die Anzahl der noch zu leistenden Verrichtungen zusätzlich als Ziffer dargestellt wird.

9. Computerprogrammprodukt mittels welchem sich das Verfahren zur Erstellung einer Zusammenfassung aus einer Patiententasche gemäß einem der Ansprüche 1 bis 8 ausführen lässt.

10. Computer mit einem Computerprogramm zur Ausführung des Verfahrens zur Erstellung einer Zusammenfassung aus einer Patiententasche gemäß einem der Ansprüche 1 bis 8.
